# FASCICULE DE BREVET EUROPEEN

(11) **EP 0 962 202 B1**
(45) Date de publication et mention de la délivrance du brevet: **30.05.2007**
(21) Numéro de dépôt: 99401352.2
(22) Date de dépôt: 04.06.1999
(51) Int. Cl.: A61F 9/00, A61K 9/48, A61M 5/178

(54) **Dispositif d'administration de fluides viscoélastiques pour chirurgie intra-oculaire**
Verabreichungsvorrichtung für viskoelastische Flüssigkeiten für intraokuläre Chirurgie
Device for administration of viscoelastic fluids for intraocular surgery

(30) Priorité: 04.06.1998 FR 9807015
(43) Date de publication de la demande: 08.12.1999
(73) Titulaire: Ioltechnologie-Production, 17000 La Rochelle (FR)
(72) Inventeur: Tourrette, Philippe, 17220 Clavette (FR)
(74) Mandataire: Santarelli

(56) Documents cités:
- EP-A- 0 444 490
- EP-A- 0 695 555
- US-A- 5 702 441
- US-A- 5 760 299

## Description

Le dispositif objet de la présente invention est du domaine des conditionnements de produits à usage chirurgical. Il concerne plus particulièrement un conditionnement spécifique de fluides utilisés en chirurgie intraoculaire, typiquement pour des opérations du segment antérieur, par exemple enlèvement de cristallin, du type général de celles pratiquées pour le traitement de problèmes de cataracte.

De façon connue, l'opération de la cataracte par extraction du cristallin nécessite une incision oculaire qui entraîne une perte de l'humeur aqueuse du segment antérieur de l'oeil. Il est alors préférable de rééquilibrer la pression de ce segment par injection d'un fluide viscoélastique qui maintient la forme tridimensionnelle de l'oeil et facilite les gestes chirurgicaux de découpe capsulaire antérieure (capsulorhexis).

Une fois la découpe capsulaire réalisée, le chirurgien effectue l'extraction du cristallin, par exemple par phacoémulsification (fragmentation par ultrasons et aspiration des fragments), puis le chirurgien procède au nettoyage du sac capsulaire par aspiration. La protection de l'endothélium cornéen est alors facilitée par la présence d'une irrigation ou d'un fluide viscoélastique.

Le chirurgien procède alors à la mise en place de l'implant formant lentille intraoculaire destiné à remplacer le cristallin opaque. A cette étape, il est favorable que le sac capsulaire soit rempli d'un autre fluide viscoélastique, qui permet par exemple à un implant souple de se déployer correctement. Par la suite, les fluides viscoélastiques sont aspirés hors de l'oeil. De telles opérations sont par exemple décrites dans le document « Experimental studies on viscofluids for intraocular surgery » (Assia, Yehezkel, Ezov, Treister, Blumenthal, J. Cataract Refract Surgery, Vol 24, 1/98 p78-83).

Lors de ces opérations, les caractéristiques chimiques ou physiques, ou la concentration des fluides viscoélastiques nécessaires ne sont pas identiques pour le maintien de la pression dans la chambre antérieure de l'oeil, notamment pendant la capsulorhexis, et pendant l'implantation de la lentille intraoculaire. Typiquement, un fluide viscoélastique de plus haute viscosité est nécessaire pendant les premières phases de l'opération, précédant la phase de phacoémulsification, pour permettre, entre autres, une meilleure protection de l'endothélium cornéen, alors qu'un fluide de plus basse viscosité est souhaitable durant l'implantation du lentille intraoculaire, notamment dans le sac cristallin, voire le succus ciliaire.

Le document EP-A-0 695 555 décrit un dispositif d'injection des médicaments, comprenant deux chambres séparées, pour l'injection des plusieurs médicaments en même temps ou pour dissoudre des médicaments solides dans un liquide avant l'injection.

Une analyse comparative des différents fluides viscoélastiques courants est fournie par le document « Les solutions viscoélastiques à usage chirurgical intraoculaire. Application à la chirurgie du segment antérieur de l'oeil. » (Lebuisson, Earith, J. Fr. Ophtalmol., 1992, 15, 2, 133-153). On peut alors citer parmi les fluides classiquement utilisés: le hyaluronate de sodium (0.7%, 1%, 2%) et en général les produits basés sur l'acide hyaluronique regroupés sous le terme générique de hyaluronan, le polyacrylamide, la méthylcellulose à 2%, la chondroitine sulfate mélangé à l'un de ces produits, un polymère carbohydrate à 2.2% ou le collagène placentaire humain type IV. Ces produits sont soit totalement synthétiques, soit d'origine animale ou végétale. Les volumes de fluides utilisés lors d'une intervention sont de l'ordre de un à quelques millilitres.

De façon courante, les chirurgiens font appel à plusieurs dispositifs d'injection de ces fluides, utilisés successivement lors de l'intervention chirurgicale. On comprend que cette situation pose des problèmes de risque de confusion de seringue autant que de simple problèmes de coût par l'existence de conditionnements spécifiques pour chaque produit.

La présente invention entend donc remédier à ces inconvénients en proposant un nouveau dispositif de conditionnement de fluides viscoélastiques pour chirurgie intraoculaire.

Selon un second objectif de l'invention, le dispositif est économique à fabriquer et donc conforme à un usage unique de ces dispositifs de conditionnement de fluides viscoélastiques.
Selon un autre objectif de l'invention, le dispositif est conforme aux préoccupations écologiques en ce qu'il réduit le nombre de conditionnement jetables.

Le dispositif objet de la présente invention est donc un dispositif d'administration de fluides viscoélastiques, comportant une seringue comprenant un réservoir, un piston et une aiguille d'injection reliée au réservoir, le réservoir étant préalablement partiellement rempli d'un premier fluide viscoélastique, caractérisé en ce que le dispositif comporte également un volume d'un second fluide viscoélastique injectable par la même aiguille.

On comprend que la présence d'un second fluide viscoélastique de caractéristiques différentes injectable par la même aiguille va permettre au chirurgien de ne pas avoir à remplacer en cours d'opération une seringue par une autre, mais au contraire de conserver le même dispositif d'injection de fluides durant toute son opération, ce qui est à la fois plus simple et plus sûr pour le patient (pas de confusion possible pour le chirurgien).

Selon une mise en oeuvre préférée, le volume de second fluide viscoélastique est disposé au sein de la même seringue, en amont du premier fluide viscoélastique.

Cette disposition est très simple de mise en oeuvre et est réalisable du fait de la différence de caractéristiques physiques des fluides viscoélastiques, qui ainsi n'ont pas tendance à se mélanger.

De façon a fournir au chirurgien une indication claire du moment ou le premier fluide viscoélastique a été complètement injecté, la seringue comporte une marque visuelle disposée en correspondance avec la position du piston lorsque la totalité du premier fluide viscoélastique a été injectée.

La description et les dessins qui suivent permettront de mieux comprendre les buts et avantages de l'invention. Il est clair que cette description est donnée à titre d'exemple, et n'a pas de caractère limitatif. Dans les dessins :
- la figure 1 représente une coupe antéro-postérieure d'un oeil ;
- la figure 2 montre un dispositif selon l'invention, à deux fluides viscoélastiques, en vue de côté ;
- la figure 3 montre une seringue contenant trois fluides viscoélastiques injectables successivement.

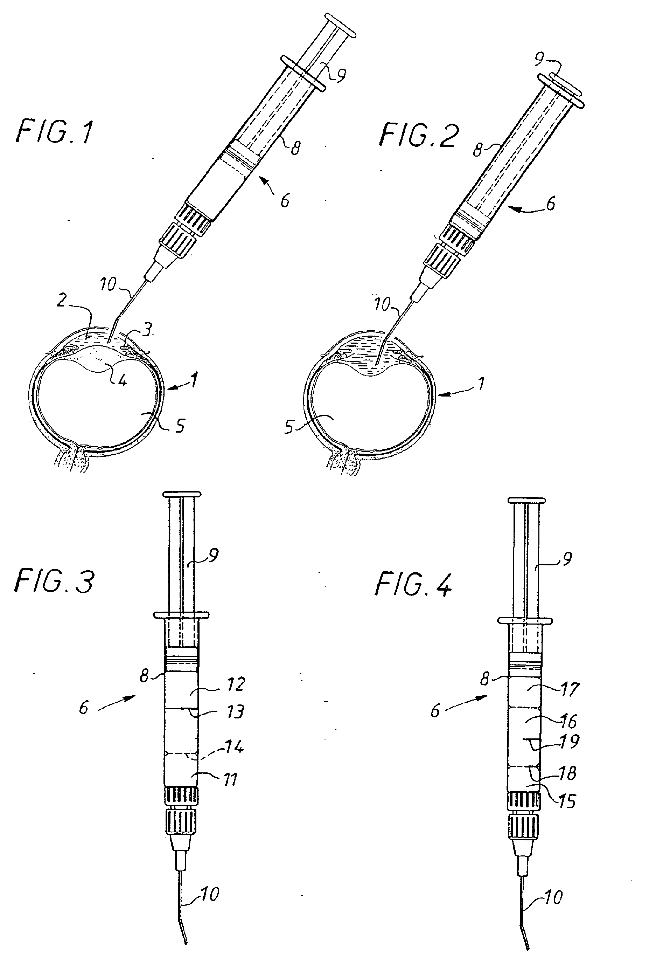

Tel que représenté sur la figure 1 selon une illustration classique, un oeil humain 1 comporte un segment (ou chambre) antérieur 2 empli d'humeur aqueuse, un iris 3, un cristallin 4 placé dans un sac capsulaire, et une chambre postérieure 5 remplie d'humeur vitrée. La dégradation de la transparence du cristallin 4 oblige à envisager son ablation et son remplacement par un implant (non représenté).

Lors de l'opération, une injection de fluide viscoélastique est réalisée à l'aide d'une seringue 6 pour rééquilibrer la pression interne dans le segment antérieur de l'oeil après l'incision sclérocornéenne, de façon à pouvoir pratiquer dans de bonnes conditions la découpe sur la face antérieure du sac capsulaire (capsulorhexis) à l'aide d'un instrument chirurgical 7 non détaillé ici. Ce premier fluide viscoélastique est ensuite évacué pendant la phacoémulsification (destruction du cristallin par ultrasons et aspiration des fragments).

Telle qu'on la voit alors sur la figure 2 en vue de côté, une seringue 6 d'injection de fluides viscoélastiques selon l'invention comporte un réservoir 8, un piston 9 et une aiguille d'injection 10 de type connu, adaptés ici à une injection de quelques dixièmes de millilitre à quelques millilitres de fluide viscoélastique. La composition exacte de la seringue n'est pas l'objet de la présente invention et ne sera donc pas détaillée plus avant ici. Il s'agit de seringue à fluide viscoélastique classique.

Cette seringue contient deux fluides viscoélastiques 11, 12 juxtaposés, et dans la forme de réalisation l'un au-dessus de l'autre, le remplissage étant effectué successivement et individuellement pour chaque fluide viscoélastique. Ce remplissage aura lieu techniquement selon des modalités habituelles en la matière. La séparation entre les fluides est figurée par la ligne 13 sur la figure 2.

A titre d'exemple non limitatif, le premier fluide viscoélastique (par exemple pour une phase de maintien du volume de la chambre antérieure et de phacoémulsification) peut être du hyaluronate de sodium 1 % et le second fluide viscoélastique (par exemple pour une phase de mise en place de l'implant) peut être du hyaluronan ou du polyacrylamide ou un mélange à base de chondroitine sulfate. Un volume de 0,5 cc du premier fluide et de 0,2 cc du second fluide est envisageable à titre d'exemple non limitatif.

Une marque 14, ici une ligne circulaire effectuée sur la seringue par tout moyen classique, signale au chirurgien que lorsque la position du bas du piston correspond à cette ligne, la totalité du premier fluide viscoélastique a été éjecté, et que c'est alors le second fluide viscoélastique qui va être éjecté. Cette marque assure donc une fonction de sécurité pour l'utilisateur.

Selon un premier aspect préférentiel de l'invention, le premier fluide viscoélastique 11 est de poids moléculaire supérieur au poids moléculaire du second fluide viscoélastique 12.

Selon un autre aspect préférentiel de l'invention, le premier fluide viscoélastique 11 est de poids moléculaire inférieur au poids moléculaire du second fluide viscoélastique 12.

Dans une variante illustrée figure 3, trois fluides viscoélastiques 15, 16, 17 sont juxtaposés dans le réservoir 8, selon des caractéristiques de nature et de volume correspondant à un certain type d'opération intraoculaire. En effet, le chirurgien pourra pour des modalités particulières d'un certain type d'intervention, souhaiter avoir à sa disposition trois, voire davantage, segments de fluide viscoélastique, qui seront chargés dans la seringue dans l'ordre désiré correspondant au déroulement de l'intervention, donc avec une très grande facilité pour le chirurgien et avec une réduction de coût grâce à ce conditionnement unique, et également une diminution du risque de confusion de seringue lors de l'intervention. La seringue comportera alors plusieurs marquages visuels 18, 19 (un de moins que le nombre de segments dans la seringue).

La porte de la présente invention ne se limite pas aux détails des formes de réalisation ci-dessus considérées à titre d'exemple, mais s'étend au contraire aux modifications à la portée de l'homme de l'art.

## Revendications

1. Dispositif d'administration de fluides viscoélastiques, comportant une seringue (6) comprenant un réservoir (8), un piston (9) et une aiguille (10) d'injection reliée au réservoir (8), le réservoir (8) étant préalablement partiellement rempli d'un premier fluide viscoélastique (11), le dispositif comportant également un volume d'un second fluide viscoélastique (12) injectable par la même aiguille (10).

2. Dispositif selon la revendication 1, **caractérisé en ce que** le volume de second fluide viscoélastique (12) est disposé au sein du même réservoir (8), en amont du premier fluide viscoélastique (11).

3. Dispositif selon la revendication 2, **caractérisé en ce que** le premier fluide viscoélastique (11) est de poids moléculaire supérieur au poids moléculaire du second fluide viscoélastique (12).

4. Dispositif selon la revendication 2, **caractérisé en ce que** le premier fluide viscoélastique (11) est de poids moléculaire inférieur au poids moléculaire du second fluide viscoélastique (12).

5. Dispositif selon l'une quelconque des revendications 2 à 4, **caractérisé en ce que** la seringue (6) comporte une marque visuelle (14) disposée en correspondance avec la position du bas du piston (9) lorsque la totalité du premier fluide viscoélastique (11) a été injectée.

## Claims

1. Device for administration of viscoelastic fluids, including a syringe (6) comprising a reservoir (8), a piston (9) and an injection needle (10) connected to the reservoir (8), the reservoir (8) being partially filled beforehand with a first viscoelastic fluid (11), the device also including a volume of a second viscoelastic fluid (12) injectable by the same needle (10).

2. Device according to claim 1, **characterised in that** the volume of second viscoelastic fluid (12) is disposed in the same reservoir (8), upstream of the first viscoelastic fluid (11).

3. Device according to claim 2, **characterised in that** the first viscoelastic fluid (11) has a molecular weight higher than the molecular weight of the second viscoelastic fluid (12).

4. Device according to claim 2, **characterised in that** the first viscoelastic fluid (11) has a molecular weight lower than the molecular weight of the second viscoelastic fluid (12).

5. Device according to any one of claims 2 to 4, **characterised in that** the syringe (6) includes a visual mark (14) disposed in correspondence with the position of the bottom of the piston (9) when all of the first viscoelastic fluid (11) has been injected.

## Patentansprüche

1. Vorrichtung für die Verabreichung von viskoelastischen Fluiden, die eine Spritze (6) mit einem Vorratsbehälter (8), einem Kolben (9) und einer mit dem Vorratsbehälter (8) verbundenen Injektionsnadel (10) umfasst, wobei der Vorratsbehälter (8) im Voraus teilweise mit einem ersten viskoelastischen Fluid (11) gefüllt worden ist, wobei die Vorrichtung außerdem ein Volumen eines zweiten viskoelastischen Fluids (12) aufweist, das durch dieselbe Nadel (10) injiziert werden kann.

2. Vorrichtung nach Anspruch 1, **dadurch gekennzeichnet, dass** das Volumen des zweiten viskoelastischen Fluids (12) innerhalb des Vorratsbehälters (8) stromaufseitig von dem ersten viskoelastischen Fluid (11) angeordnet ist.

3. Vorrichtung nach Anspruch 2, **dadurch gekennzeichnet, dass** das erste viskoelastische Fluid (11) ein Molekulargewicht hat, das höher als das Molekulargewicht des zweiten viskoelastischen Fluids (12) ist.

4. Vorrichtung nach Anspruch 2, **dadurch gekennzeichnet, dass** das erste viskoelastische Fluid (11) ein Molekulargewicht hat, das niedriger als das Molekulargewicht des zweiten viskoelastischen Fluids (12) ist.

5. Vorrichtung nach einem der Ansprüche 2 bis 4, **dadurch gekennzeichnet, dass** die Spritze (6) eine visuelle Markierung (14) aufweist, die in Übereinstimmung mit der Position der Unterseite des Kolbens (9) angeordnet ist, wenn das gesamte erste viskoelastische Fluid (11) injiziert worden ist.
